# EUROPÄISCHE PATENTSCHRIFT

(11) **EP 1 311 238 B1**
(45) Veröffentlichungstag und Bekanntmachung des Hinweises auf die Patenterteilung: **21.10.2009**
(21) Anmeldenummer: 01976087.5
(22) Anmeldetag: 16.08.2001
(51) Int. Cl.: A61K 8/14, A61K 8/55, A61K 31/683, A61Q 5/00, A61Q 17/04, A61Q 19/00

(54) **VERWENDUNG VON DI-ZUCKERALKOHOLPHOSPHATEN ZUM SCHUTZ MENSCHLICHER HAUTZELLEN VOR UV- UND/ODER IR-STRAHLUNG**
USE OF DI-SUGAR ALCOHOL PHOSPHATES TO PROTECT HUMAN SKIN CELLS FROM UV-AND/OR IR RADIATION
UTILISATION DE DI-SUCRES DE PHOSPHATE D'ALCOOL POUR PROTEGER LES CELLULES DE LA PEAU HUMAINE CONTRE LES RADIATIONS UV ET/OU IR

(30) Priorität: 18.08.2000 DE 10040932; 22.08.2000 DE 10055706
(43) Veröffentlichungstag der Anmeldung: 21.05.2003
(73) Patentinhaber: Bitop Gesellschaft für Biotechnische Optimierung mbH, 58453 Witten (DE)
(72) Erfinder: SCHWARZ, Thomas, 42799 Leichlingen (DE)
(74) Vertreter: Thiel, Christian
(86) Internationale Anmeldenummer: PCT/EP2001/009446
(87) Internationale Veröffentlichungsnummer: WO 2002/015868

(56) Entgegenhaltungen:
- EP-A- 0 965 268
- EP-A- 1 125 583
- WO-A-00/76528
- WO-A-01/76572
- PT-A- 101 813
- LAMOSA P ET AL: "THERMOSTABILIZATION OF PROTEINS BY DIGLYCEROL PHOSPHATE, A NEW COMPATIBLE SOLUTE FROM THE HYPERTHERMOPHILE ARCHAEOGLOBUS FULGIDUS" APPLIED AND ENVIRONMENTAL MICROBIOLOGY, WASHINGTON,DC, US, Bd. 66, Nr. 5, Mai 2000 (2000-05), Seiten 1974-1979, XP001034354 ISSN: 0099-2240
- SCHOLZ S ET AL: "DI-MYO-INOSITOL-1,1'-PHOSPHATE: A NEW INOSITOL PHOSPHATE ISOLATED FROM PYROCOCCUS WOESEI" FEBS LETTERS, ELSEVIER SCIENCE PUBLISHERS, AMSTERDAM, NL, Bd. 306, Nr. 2/3, 20. Juli 1992 (1992-07-20), Seiten 239-242, XP001030747 ISSN: 0014-5793

## Beschreibung

Die Erfindung betrifft kosmetische und dermatologische Formulierungen, Zubereitungen oder Produkte.

### Stand der Technik

### UV- und IR-Schutz

Heutige Sonnenschutzmittel beinhalten zwei Klassen von UV-Filtern. Physikalisch wirksame UV-Filter bestehen aus anorganischen Verbindungen (z. B. Titandioxid), die das auftreffende Licht auf der Haut reflektieren. Chemisch wirkende organische Moleküle absorbieren UV-Licht der Wellenlängen, die für die Ausbildung von Erythemen (Sonnenbrand) verantwortlich sind (vornehmlich 295 bis 323 nm mit einem Maximum bei 308 nm).

Somit sind alle bisherigen Sonnenschutzpräparate reine "Sonnenbrandverminderer", die nur dem dramatischen, für den Menschen leicht ersichtlichen Effekt der Hautschädigung vorbeugen. In der Literatur wird aber bereits vor den wesentlich gefährlicheren Zellschädigungen gewarnt, die im Vorfeld eines Erythems wirksam werden, wie der massiven DNA-Schädigung, die bis zur Ausbildung eines Melanoms entarten kann.

### Radikalfänger und Antioxidantien

Im Bereich der Kosmetik finden eine Vielzahl von Radikalfängern und Antioxidantien als Zusatzstoffe in kosmetischen Formulierungen ihre Anwendung zum Schutz vor oxidativem Stress, der durch Umwelteinflüsse (UV-Strahlung, Rauch, chemische Substanzen) aber auch durch zelleigene Aktivitäten entsteht. Die Wirkstoffpalette deckt sowohl wasserlösliche Radikalfänger (z. B. Ascorbinsäure), die im flüssigen Zellinneren wirken, wie auch fettlösliche Antioxidantien (z. B. Retionolpalmitat oder α-Tocopherol), die in Membranstrukturen wirksam werden, ab. Dabei wird nur selten bedacht, daß bei topischen Applikationen dieser Wirkstoffe höchstens 3 % eine tiefere Hautschicht erreichen und der Rest unwirksam an der Hautoberfläche verbleibt.

### Substanzpenetration in die menschliche Haut; Cosolventien

Die menschliche Haut besteht zum Schutz vor schädigenden Umwelteinflüssen aus mehreren Schichten (Stratum comeum, Subdermis, Dermis, Epidermis), die eine praktisch undurchdringliche Barriere für Fremdstoffe darstellt. Mögliche Penetrationswege für kosmetische Wirkstoffe sind interzellulär (nur für kleine polare und unpolare Stoffe durch laterale Diffusion durch die hochorganisierten interzellulären Lipidschichten möglich), intrazellulär oder transfollikulär (Liposomentechnologie). Nachweislich gelangen jedoch selbst mit der fortschrittlichen Liposomenverkapselung in Nanopartikel kosmetische Wirkstoffe nur zu einem extrem geringen Prozentsatz (<3 %) durch die oberen Hautschichten zu ihrem eigentlichen Zielbestimmungsort, wo sie ihre schützende Wirkung erst entfalten sollen. Größere Moleküle, wie die in ihrer potentiellen Wirkung vielversprechenden Enzyme (z. B. Sericin, Kollytin, Glutathion-S-Transferase oder die Gruppe der Cytokinen), sind durch die sterische Hinderung praktisch selbst mit modernsten Methoden nur minimal In lebende Zellen einzuschleusen.

Angesichts dieser Problematik besteht Bedarf an neuartigen Formulierungen mit verbesserter kosmetischer Wirksamkeit in vorgenannten Einsatzgebiet des UV- und IR-Schatzer.

Diese Aufgabe wird erfingdungsgemäß gelöst durch Formulierungen gemäß Anspruch 6 und eine Verwendung nach Anspruch 1. Für diese Zwecke besonders geeignete Formulierungen sind Gegenstand der abhängigen Ansprüche 7 bis 10.

Es sind bisher keine Substanzen in der präventiven Sonnenkosmetik bekannt, die die zelleigenen Reparaturmechanismen der Zelle aktiv unterstützen und so "von innen" die Zelle vor erhöhter UV-Strahlung schützen. Durch den nachgewiesenen DNA-Schutz und die proteinstabilisierende Wirkung stellt der erfindungsgemäße Einsatz von Di-Zuckeralkoholphosphaten von C₃ bis C₆ Zuckeralkoholen eine neue Art des aktiven UV-Sonnenschutzes in der Kosmetik dar.

Überraschenderweise wurde nun gefunden, dass IR-Strahlung zu thermischen Schädigungen von Hautzellen und zur Denaturierung von Zellproteinen und -enzymen führt. In der Kosmetikindustrie sind keine Wirkstoffe bekannt, die vor Zellstress durch erhöhte Temperatur schützen. Extremophile Mikroorganismen schützen vor thermischem Stress durch die Bildung kompatibler Solute wie DIP oder DGP. Deshalb ist ein effektiver Einsatz dieser Substanzklasse in diesem noch neuen Anwendungsbereich zu sehen.

Grundlage der Erfindung ist der überraschende Befund, daß die Di-Zuckeralkoholphosphate von C₃ bis C₆ Zuckeralkoholen, nämlich die kompatiblen Solute DIP und DGP und/oder Derivate dieser Verbindungen, z. B. Säuren, Salze oder Ester, ein umfassendes Wirkungsspektrum für den Einsatz in kosmetischen oder dermatologischen Formulierungen zeigen. Die Zuckeralkoholreste der erfindungsgemäßen Di-Zuckeralkoholphosphate sind Alkohole von C₃ bis C₆ Zuckern und können linear oder cyclisch, modifiziert oder unmodifiziert sein. Dabei können die beiden Zuckeralkoholreste eines Di-Zuckeralkoholphosphats jeweils gleich- oder verschiedenartig sein.

Jedes Gewebe besitzt ein antioxidatives Potential (AOP) aus enzymatischen und nicht-enzymatischen Antioxydantien, die bei ungestressten Zellen den Gehalt von Prooxidantien unterhalb eines Grenzwertes hatten, der für die gesunde Zelle unschädlich ist. Die Applikation von Di-Zuckeralkoholphosphaten von C₃ bis C₆ Zuckeralkoholen unterstützt durch die nachgewiesene Proteinstabilisierung das zelleigene antioxidative Potential gemäß dieser Erfindung. Besonders wirksam sind hier die als kompatible Solute in extremophilen Mikroorganismen identifizierten Substanzen Di-myo-Inositolphosphat (DIP) und Di-Glycerinphosphat (DGP).

DIP und DGP sind grundsätzlich im Stand der Technik bereits bekannt, jedoch zu anderen Zwecken. In der EP 0 965 268 A1 wird die Verwendung von DGP als Stabilisator für Enzyme und andere Zellkomponenten gezeigt.

Aus der Veröffentlichung P. Lamosa et al.: "Thermostabilization of Proteins by Diglycerol Phosphate, a New Compatible Solute from the Hyperthermophile *Archaeoglobus fulgidus*", *Applied and Environmental Microbiology* **2000,** *66*, 1974-1979 ist ein stabilisierender Effekt von DGP auf verschiedene Enzyme wie LDH, ADH, GDH oder Rubredoxin bekannt. Die portugiesische Patentanmeldung PT 101 813 A schließlich beschreibt die Verwendung von DIP unter anderem zur Stabilisierung von Liposomen.

Durch ihre Herkunft aus extremophilen Mikroorganismen, die sich durch die Produktion von den erwähnten kompatiblen Soluten vor umweltbedingten Stresssituationen, wie UV-Strahlung, Wärme, Kälte, Trockenheit oder osmotischem oder chemischem Stress, effektiv schützen und nur auf diese Weise überleben, stellen insbesondere Substanzklassen der DIP und DGP einen innovativ neuen Weg für die Bekämpfung von schädigenden Umwelteinflüssen auf die menschliche Haut dar.

Zelleigene Reparatursysteme (DNA-Reparatur nach UV-Schädigung, Induktion und Stabilisierung der chaperon-Proteine zur Neufaltung (teil-) denaturierter Proteine und Enzyme) können erstmals durch die DIP und DGP enthaltenden Formulierungen, Zubereitungen und Produkte effektiv "von innen" (in vivo) stabilisiert und aktiviert werden. Die Aufrechterhaltung des antioxidativen Potentials der Zelle, ausgelöst durch Protein- und DNA-Schädigung durch Strahlung, freie Radikale oder Lipidoxidation der Zellmembranen wird durch DIP und DGP gewährleistet.

Durch das geringe Molekulargewicht gelangen die erfindungsgemäßen Di-Zuckeralkoholphosphate durch alle Hautschichten bis ins Innere der Zielzellen und können als Co-Solvenz oder Penetrationsverstärker andere Wirkstoffe aus der kosmetischen Formulierung (z. B. Proteine, Enzyme, Vitamine, Antioxydantien) mit an den eigentlichen Bestimmungsort bringen.

Aufgrund ihrer chemischen Struktur sind die erfindungsgemäßen Di-Zuckeralkoholphosphate DIP oder DGP mit der Mehrzahl der üblichen kosmetischen Grundformulierungen verträglich, und durch entsprechende Modifikationen der funktionellen Seitenketten sind weitere Anpassungen an die geeignete Applikationsform möglich. Dabei wirken die Di-Zuckeralkoholphosphate durch die Vielzahl der Hydroxygruppen feuchtigkeitsspendend und vermitteln ein angenehmes Hautgefühl.

Kompatible Solute wirken als natürliche Lösungsmittel in extremophilen Mikroorganismen und stabilisieren Enzyme, Proteine und andere Zellbestandteile durch die Wiederherstellung eines nativen, globulären Zustandes, der die Proteine nicht nur vor Proteolyse schützt, sondern diese auch in einem kompakteren und aktiveren Zustand hält. Als Co-Solvens oder Penetrationsverstärker für kosmetische Wirkstoffe sind DIP und DGP besonders im Hinblick auf eine mögliche Derivatisierung einzelner funktioneller Gruppen in einem weiten Feld einsetzbar.

Der positive Einfluß der kompatiblen Solute DIP und DGP in kosmetischen und dermatologischen Formulierungen konnte mit einer Reihe von anerkannten in-vivo- und in-vitro-Testverfahren gezeigt werden.

Mit Zellkulturen läßt sich in-vitro der Effekt von DIP und DGP sowie deren Derivaten auf die verschiedensten Umwelteinflüsse, insbesondere UV- und IR-Strahlung einfach darstellen. Das antioxidative Potential von Gewebe kann mittels Elektronenspinresonanzmessung durch Ermittlung des RPF ("Radical Protection Factor") bestimmt werden.

Mit der nicht-invasiven Methode der Biophotonik kann der Einfluß externer Behandlungen auf temporale und strukturelle Änderungen der Haut als Reaktion der lebenden Zellen oder des Gewebes auf Streß oder Heilungsprozesse direkt beobachtet werden.

Das Schweinehautmodell und Versuche an menschlicher Haut wurden schließlich für Versuche zum Substanzpenetrationsverhalten durch einzelne Hautschichten herangezogen.

### Ergebnisse

Die hervorragenden Eigenschaften von Di-myo-Inositolphosphat bei der Stabilisierung von Proteinen und Enzymen unter verschiedenen Streßbedingungen und die strukturelle Verwandtschaft zu den elementaren Bausteinen von Zellmembran und Membranen verschiedener Zellorganellen machen dieses kompatible Solut besonders für den Einsatz im Bereich der Kosmetik interessant.

Proteine und Peptide als Wirkstoffe gewinnen auch in der Kosmetik/Dermatologie eine immer größere Bedeutung. Beispiele für derartige Applikationen in der Kosmetik sind z. B. Kollagenpräparationen, der Einsatz von Cytokinen bei antientzündlich wirkenden Präparaten, der Schutz des sog. Mikroproteins oder auch von Reparaturenzymen der Haut.

Die hautverträgliche Stabilisierung und Erhaltung der Funktionalität dieser Präparate gegenüber Temperaturdenaturierung und Proteolyse ist entscheidend für den Anwendungserfolg. Die funktionale Stabilisierung von Proteinen und Peptiden in den Präparationen (Lagerung, Transport) aber auch nach der Applikation auf der Haut (Depotwirkung) ist von großer Bedeutung. Insbesondere auf der Haut sind viele "proteinfeindliche" Abwehrmechanismen installiert (z. B. Proteasen), die den Erfolg einer proteingestützten kosmetischen Applikation gefährden.

In Experimenten konnten die hervorragenden Eigenschaften von Di-myo-Inositolphosphat bei der Stabilisierung von Proteinen und Enzymen unter verschiedenen Streßbedingungen gezeigt werden.

Denaturierungs- und damit kausal verbundene Alterungsprozesse verlaufen immer nach einer Funktion, die von den Faktoren Zeit und Temperatur abhängig ist. Temperaturbedingte Denaturierung läuft somit nicht nur bei außergewöhnlich hohen Temperaturen, sondern auch bei physiologischen Temperaturen ab. Jede Steigerung der Temperaturstabilität bedeutet somit eine Verlangsamung von Denaturierungs- und Alterungsprozessen. Wirkstoffe die auf diese Weise gegen das sogenannte "skin-aging" schützen sind für die Kosmetikindustrie von enormem Interesse.

Di-myo-Inositolphosphat zeigte in Experimenten eine starke Wasserbindungsfähigkeit und hohe Stabilität, die durch die ausgeprägte Neigung der zahlreichen Hydroxygruppen im Molekül zur Ausbildung von Wasserstoffbrücken gekennzeichnet ist. Die Verbindung ist somit zusätzlich als Regulator des Feuchtigkeitshaushaltes der Haut von Interesse. Dies ist zumindest ein zusätzlicher Vorteil beim Einsatz der Verbindung als Kosmetikwirkstoff.

Austrocknungsprozesse schädigen Hautbestandteile irreversibel. Zellen, Membranen und Proteine werden zerstört. Die Widerstandskraft der Haut und deren Regenerationsfähigkeit nimmt ab. Hautalterungsprozesse laufen letztendlich beschleunigt ab. Während der Gefriertrocknung läuft im Prinzip der gleiche Prozeß wie bei der Austrocknung der Haut ab, die Streßbedingungen sind nur noch viel größer. Aus dem Schutzpotential des Di-myo-Inositolphosphats bei der Gefriertrocknung kann daher zumindest plausibel abgeleitet werden, daß Di-myo-Inositolphosphat bei Trocknungsprozessen mehr als nur ein Feuchtigkeitsgeber, sondern auch ein aktiver Schutzstoff sein kann.

### Strukturformel von Di-myo-Inositolphosphat

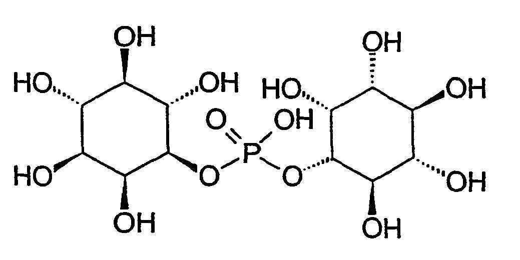

Mit Di-Glycerinphosphat (DGP) wurden Anwendungsuntersuchungen zum Schutz von Modellproteinen durchgeführt. Als Penetrationsverstärker sorgt Di-Glycerinphosphat weiterhin dafür, daß diese Makromoleküle auch bis an ihren Zielort durch mehrere Hautschichten transportiert werden. In den Versuchen konnte eindeutig gezeigt werden, daß Di-Glycerinphosphat ein wesentlich besseres Schutzpotential als z. B. das in der Kosmetik häufig verwendete Konservierungsmittel Glycerin hat.

### Strukturformel von Di-Glycerinphosphat

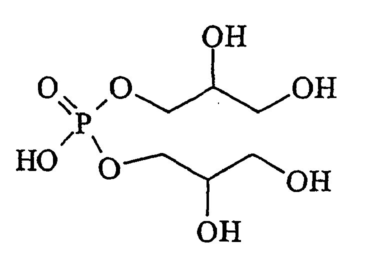

Gegenstand der Erfindung ist die Verwendung einer oder mehrerer chemischen Verbindungen ausgewählt aus den Verbindungen des Anspruchs 1 in kosmetischen und dermatologischen Formulierungen. Die Erfindung betrifft z. B. die Herstellung einer kosmetischen oder dermatologischen Formulierung zum Schutz und zur Stabilisierung der menschlichen Hautzellen sowie deren organischer Bestandteile wie z. B. Proteine, Enzyme, Membranen, Nukleinsäuren oder Antioxidantien. Die Formulierungen enthalten mindestens eine der nachfolgend dargestellten Verbindungen Ia und Ib:

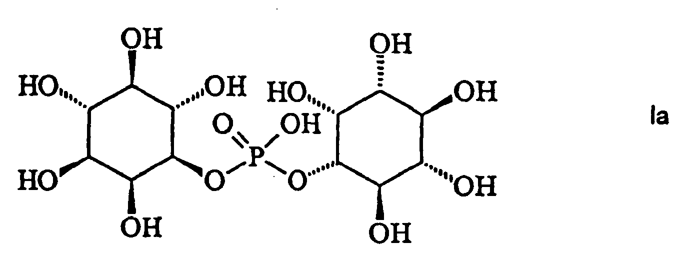

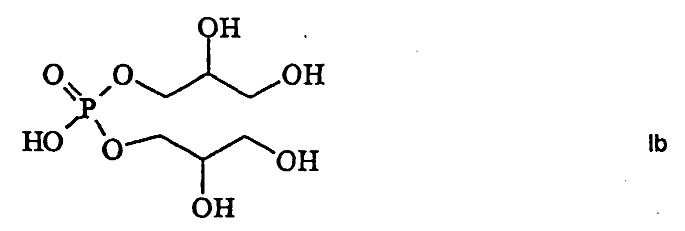

Die Erfindung schließt die Verwendung von physiologisch verträglichen Salzen der Verbindungen Ia und Ib, den stereoisomeren Formen dieser Verbindungen und Derivaten dieser Verbindungen (z. B. Ester) zur Verwendung in kosmetischen und dermatologischen Formulierungen mit ein. Als Salze kommen Alkali- und Erdalkalisalze, insbesondere aber das Na- und K-Salz in Frage. Als Ester können grundsätzlich alle physiologisch verträglichen Ester eingesetzt werden.

Durch ihre Herkunft aus extremophilen Mikroorganismen, die sich durch die Produktion von neuartigen kompatiblen Soluten wie Diinositolphosphat vor umweltbedingten Stresssituationen, wie UV-Strahlung, Wärme, Radikalen, Kälte, Trockenheit oder osmotischem oder chemischem Stress, effektiv schützen und nur auf diese Weise überleben, stellen diese Substanzen einen völlig neuen Weg zum Schutz von Organismen und deren Komponenten vor schädigenden Umwelteinflüssen dar.

Zelleigene Reparatursysteme (DNA-Reparatur nach UV-Schädigung, Induktion und Stabilisierung der chaperon-Proteine zur Neufaltung (teil-)denaturierter Proteine und Enzyme) können erstmals durch DIP und DGP stabilisiert und aktiviert werden. Die Aufrechterhaltung des antioxidativen Potentials der Zelle (Protein- und DNA-Schädigung durch freie Radikale, Lipidoxidation der Zellmembranen) wird erstmals durch DIP und DGP gewährleistet.

Durch ihr geringes Molekulargewicht gelangen DIP und DGP durch mehrere Hautschichten bis ins Innere der Zielzellen und können als Co-Solventien oder Penetrationsverstärker andere Wirkstoffe aus der kosmetischen Formulierung (z. B. Proteine, Enzyme, Vitamine, Antioxydantien) mit an den eigentlichen Bestimmungsort bringen.

Aufgrund ihrer chemischen Struktur sind insbesondere DIP und DGP mit der Mehrzahl der üblichen kosmetischen Grundformulierungen verträglich und durch entsprechende Modifikationen der funktionellen Seitenketten sind weitere Anpassungen an die geeignete Applikationsform möglich. Dabei wirkt Diinositolphosphat durch die Vielzahl der Hydroxygruppen feuchtigkeitsspendend und vermitteln ein angenehmes Hautgefühl.

Gegenstand der Erfindung ist somit die Verwendung von DIP und/oder DGP als Additive und Wirkstoffe mit einem völlig neuartigen Wirkprinzip in kosmetischen und dermatologischen Präparaten und Produkten.

### Kompatible Solute und Hyperthermophile

Hyperthermophile sind sehr außergewöhnliche Mikroorganismen, da sie optimal bei Temperaturen (60-110°C) wachsen, die bei mesophilen ("normalen") Organismen zu massiven Schädigungen zellulärer Strukturen führen würden. In den letzten Jahren wurde daher ein großer Forschungsaufwand betrieben, um die biochemischen Komponenten zu identifizieren, die zu der bemerkenswerten thermischen, chemischen und physikalischen Stabilisierung der Zellstrukturen führt. Der Schwerpunkt der Forschungsarbeiten lag hierbei auf der Isolierung thermostabiler Enzyme, da zahlreiche enzymatisch katalysierte industrielle Prozesse unter extremen Milieubedingungen ablaufen und hierfür geeignete Biokatalysatoren gesucht werden.

Obwohl viele Enzyme aus hyperthermophilen Mikroorganismen auch unter hohen Temperaturen stabil sind, gilt dies nicht generell für die zellulären Strukturen thermo- und hyperthermophiler Organismen. Zu der hohen Temperaturstabilität von Zellstrukturen tragen in erheblichem Maß niedermolekulare organische Substanzen (Kompatible Solute, "Hypersolutes") im intrazellulären Milieu bei. Verschiedene neuartige Hypersolutes konnten in den letzten Jahren in hyperthermophilen Mikroorganismen erstmals identifiziert werden. In einigen Fällen konnte der Beitrag dieser Verbindungen zum Schutz zellulärer Strukturen - vor allem Enzymen - gegenüber Hitze- und Trockenheit bereits eindrucksvoll gezeigt werden. bitop hat Technologien zur Produktion kompatibler Solute aus thermophilen Mikroorganismen entwickelt.

Die Haut ist als Grenzschicht und Oberfläche des menschlichen Körpers einer Vielzahl externer Stressfaktoren ausgesetzt. Die Human-Haut ist ein Organ, das mit verschiedenartig spezialisierten Zelltypen - den Keratinozyten, Melanozyten, Langerhanszellen, Merkel-Zellen und eingelagerten Sinneszellen - den Körper vor äußeren Einflüssen schützt. Hierbei ist zwischen äußeren physikalischen, chemischen und biologischen Einflüssen auf die menschliche Haut zu unterscheiden. Zu den äußeren physikalischen Einflüssen sind thermische und mechanische Einflüsse sowie die Einwirkung von Strahlung, z. B. UV-, VIS, und IR-Strahlung, zu zählen. Unter den äußeren chemischen Einflüssen sind insbesondere die Einwirkung von Toxinen, Allergenen und Substanzen, die an die Desoxyribonukleinsäure anbinden, zu verstehen. Die äußeren biologischen Einflüsse umfassen die Einwirkung fremder Organismen und deren Stoffwechselprodukte.

Eine übermäßige Sonnenstrahlung führt sowohl zu akuten Hautschäden wie beispielsweise Sonnenbrand als auch zu chronischen Veränderungen wie z. B. Hautalterung oder Hautkrebs. Der Sonnenbrand (Erythema solare) entwickelt sich überwiegend als Folge der UV-B-Bestrahlung. Die UV-A-Strahlung hat dagegen einen vergleichsweise geringen Einfluß auf seine Entstehung. Der Sonnenbrand kann von einer leichten Tötung bis hin zu einer starken Verbrennung mit Blasenbildung auftreten. Da diese Folgen frühestens 4-6 h nach der Bestrahlung auftrete, ist es für Gegenmaßnahmen zu spät. Mehrere Sonnenbrände - ganz besonders in der Kindheit - erhöhen deutlich das Hautkrebsrisiko. Ursachen hierfür sind Schädigungen, insbesondere der Nukleinsäuren von menschlichen Hautzellen und eine fehlerhafte Reparatur der geschädigten Desoxyribonukleinsäure im Zellkern sowie wahrscheinlich die immunsuppressive Wirkung der UV-Strahlung, d.h. die Schwächung der Immunreaktion durch UV-Bestrahlung. Die übermäßige UV-A- und UV-B-Exposition trägt zur Hautalterung bzw. Lichtalterung bei, z. B. in Form von strukturellen Veränderungen des Bindegewebes (aktinische Elastose). Die übermäßige UV-B-Exposition ist die wesentliche Ursache für chronische Hautveränderungen.

Aufgrund eines veränderten Freizeitverhaltens, wie z. B. ausgiebiges Sonnenbaden oder Femreisen in Ländern mit einer starken Sonneneinstrahlung, sind die Gefahren einer UV-Schädigung der Hautzellen in den letzten Jahren stark angestiegen, was sich wiederum in einer Erhöhung des Hautkrebsrisikos niederschlägt. Zusätzlich ist das Hautkrebsrisiko in jüngerer Zeit durch eine zunehmende UV-Strahlung an der Erdoberfläche, ausgelöst durch die Abnahme der Ozonschicht, und durch eine höhere Lebenserwartung der Menschheit deutlich angestiegen.

Neben dem weiterhin sehr aktuellen Problem der nachhaltigen Zellschädigung durch UV-Strahlung, was durch die Verwendung herkömmlicher Sonnenschutzmittel bislang nur unzureichend gelöst ist, wird der bisher unterschätzte Einfluß der IR-Strahlung im Bereich der thermischen Schädigung von Hautzellen (thermischer Stress denaturiert Zellproteine und -enzyme) in der Forschung aktuell diskutiert. In der Kosmetikindustrie sind keine Wirkstoffe bekannt, die vor Zellstress durch erhöhte Temperatur schützen.

Jedes Gewebe besitzt ein antioxidatives Potential (AOP) aus enzymatischen und nicht-enzymatischen Antioxydantien, die bei ungestressten Zellen den Gehalt von Prooxidantien unterhalb eines Grenzwertes halten, der für die gesunde Zelle unschädlich ist. Kommt es zu einer Inaktivierung oder Denaturierung dieser natürlichen Antioxydantien wird die Widerstands- und Regenerationsfähigkeit der Haut signifikant erniedrigt.

Es sind bisher nur wenige natürliche Substanzen in der präventiven Kosmetik und Dermatologie bekannt, die die zelleigenen Reparaturmechanismen der Zelle aktiv unterstützen und so "von innen" (*in vivo*) die Zelle z. B. vor erhöhter UV-Strahlung, IR-Strahlung, Wärme, Radikalen, Kälte, Trockenheit, osmotischem Stress oder chemischem Stress schützen. Es bestand daher die Aufgabe, kosmetische und dermatologische Formulierungen zur Verfügung zu stellen, deren Anwendung die obengenannten Hautprobleme beseitigen oder zumindest mindern und insbesondere zum Schutz und zur Stabilisierung von menschlichen Hautzellen und deren organischer Komponenten geeignet sind.

Somit ist es ein generelles Problem der Kosmetik und Dermatologie, dass keine chemisch eindeutig definierten und unschädlichen Wirkstoffe und Additive natürlichen Ursprungs zur Verfügung stehen, um die oben genannten wichtigen Aufgabenstellungen dieser Anwendungsbereiche zufriedenstellend zu lösen. Die vorliegende Erfindung schlägt eine Lösung dieses Problems vor.

### Erfindung / Vorteilhafte Wirkungen der Erfindung

Extremophile Mikroorganismen (Thermophile und Hyperthermophile) schützen sich vor thermischen Stress durch die Bildung von kompatiblen Soluten, insbesondere DIP und DGP. In Gegenwart dieser Substanzen ist es diesen mikrobiellen Lebewesen daher möglich unter den herrschenden Extrembedingungen zu existieren, da diese Verbindungen den Stoffwechsel und die essentiellen organischen Bestandteile stabilisierten und vor Schädigungen schützen.

Überraschenderweise wurde nun insbesondere gefunden, dass DIP und DGP die Stabilisierungseffekte auch auf der menschlichen Haut zeigen und somit ideale Wirkstoffe und Additive für kosmetische und dermatologische Präparate darstellen. Überdies zeigte sich, daß diese und ähnliche Effekte durch die Substanzen der Substanzklasse der Di-Zuckeralkoholphosphate von C₃ bis C₆ Zuckeralkoholen insgesamt bewirkt werden können. Di-Zuckeralkoholphosphate von C₃ bis C₆ Zuckeralkoholen stellen somit ein universal einsetzbares und völlig neuartiges Schutzprinzip in der Kosmetik und Dermatologie dar. Somit wird gemäß der Erfindung das o.g. Problem durch die Verwendung von einer oder mehrerer Verbindungen in kosmetischen und dermatologischen Formulierungen gelöst. Die Erfindung betrifft zudem die Verwendung von Verbindungen der Formeln Ia (Diinositolphosphat), 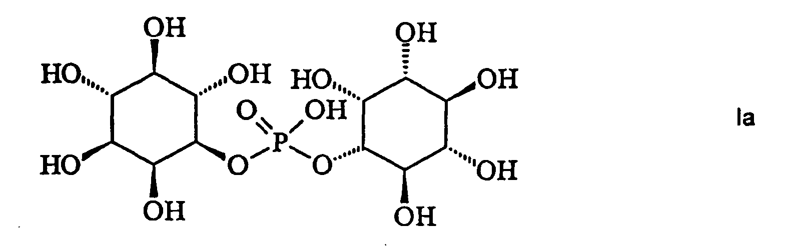 den physiologisch verträglichen Salzen der Verbindung Ia, den stereoisomeren Formen der Verbindungen der Formel Ia sowie von Derivaten zur Herstellung einer kosmetischen oder dermatologischen Formulierung zum Schutz und zur Stabilisierung der menschlichen Hautzellen sowie deren organischer Bestandteile wie z. B. Proteine, Enzyme, Membranen, Nukleinsäuren oder Antioxidantien. Bevorzugt sind weiterhin Verbindungen der Formel Ib (Di-Glycerinphosphat), 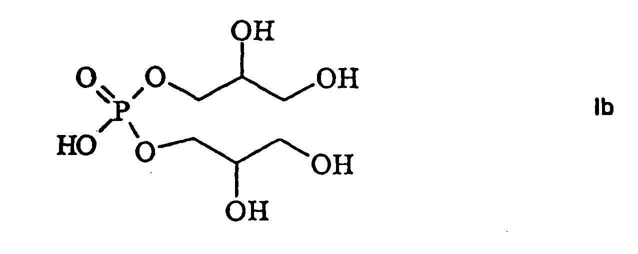 den physiologisch verträglichen Salzen der Verbindung Ib, den stereoisomeren Formen der Verbindungen der Formel Ib sowie von Derivaten zur Herstellung einer kosmetischen oder dermatologischen Formulierung zum Schutz und zur Stabilisierung der menschlichen Hautzellen sowie deren organischer Bestandteile wie z. B. Proteine, Enzyme, Membranen, Nukleinsäuren oder Antioxidantien.

Die Erfindung betrifft weiterhin die Verwendung einer oder mehrerer der vorgenannten Verbindungen, deren physiologisch verträgliche Salze und/oder stereoisomere Formen zur Herstellung einer kosmetischen oder dermatologischen Formulierung zum Schutz und zur Stabilisierung der menschlichen Hautzellen sowie deren organischer Bestandteile wie z. B. Proteine, Enzyme, Membranen, Nukleinsäuren, Antioxidantien usw. vor physikalischen, chemischen und biologischen Einflüssen wie z. B. vor Strahlung (UV, IR, VIS-Strahlung), vor denaturierenden Substanzen, vor Temperatur oder vor Kälte.

Die vorliegende Erfindung beschreibt dieses Schutzprinzip erstmals. Die Einsatzmöglichkeiten sind sehr vielseitig und können im Rahmen der Erfindung nur beispielhaft beschrieben werden. Die Erfindung soll durch Nennung von Beispielen jedoch nicht eingeschränkt werden.

Membranen stellen die natürliche Barriere von Zellen und Zellorganellen zum umgebenden Medium dar. Membranen sorgen dafür, dass in den einzelnen Zellen bzw. Zellbestandteilen eine konstantes inneres Milieu besteht. Biologische Membranen sind Mehrkomponentensysteme, die im allgemeinen aus Lipiden, Cholesterol und Proteinen bestehen. Die genaue Zusammensetzung einzelner Membranen unterliegt je nach ihrer Herkunft mehr oder weniger großen Schwankungsbreiten. Elementare Bestandteile von Membranen sind jedoch Lipide und Proteine. Lipide lassen sich in die sogenannten "einfachen" Lipide wie Fette und Wachse und "komplexen" Lipide unterteilen. Als Grundbausteine von Membranen sind hierbei die komplexen Lipide wie die Glycerophospholipide (Glycerinphosphatide) am weitesten verbreitet. Die allgemeine Struktur dieser Membranbestandteile leitet sich von sn-Glycerin-3-phosphorsäure (folgende Abbildung) ab.

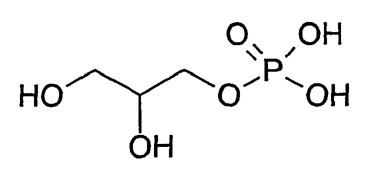

Es handelt sich dabei um Phosphorsäurediester, in denen der Glycerinrest i.a. mit gesättigten (R1) und ungesättigten (R2) Fettsäuren kondensiert ist. Der Phosphorsäurerest ist zusätzlich mit einer Komponente alkoholischer Struktur wie Inositol oder Ethanolamin (R3) kondensiert (folgende Abbildung).

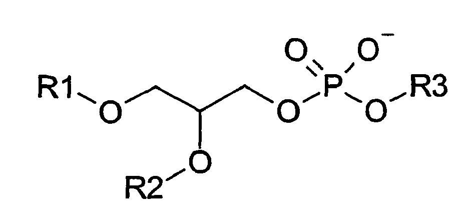

Durch ihre ambivalente Struktur mit einem hydrophilen Phosphatrest und lipophilen Fettsäureresten sind Glycerophospholipide in der Lage, in wäßriger Lösung Aggregate zu bilden (z. B. Doppelschichten oder Miccellen), bei denen der nur hydrophile Phosphatrest mit der wäßrigen Phase in Berührung kommt. Diese Struktur findet man auch in Form geschlossener lamellarer Vesikel, den sogenannten Liposomen wieder.

### DIP:

Eine besondere Klasse der Phospholipide stellen die Phosphatidylinositole dar. Es handelt sich hierbei um stickstoffreie Glycerophospholipide, bei denen die Phosphorsäureestergruppierung der Phosphatidsäure mit myo-Inositol verestert ist. Diese stellen in den subzellulären Membranen bis zu 10 % der Gesamtlipide dar. Darüber hinaus sind sie in einigen Fällen am biochemischen Informationstransport beteiligt.

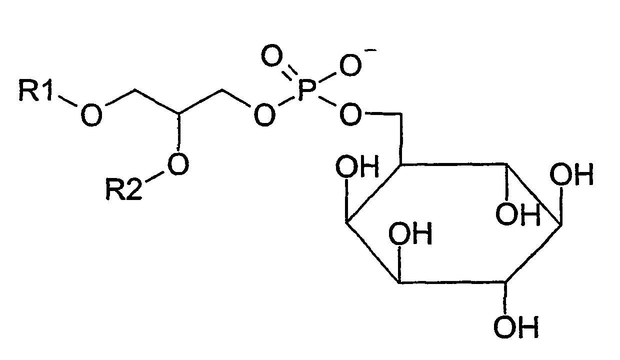

Der Vergleich des kompatiblen Solutes Di-myo-Inositolphosphat (DIP) mit den zuvor beschriebenen Glycerophospholipiden zeigt deutlich die strukturelle Nähe der Verbindungen. Im Falle von DIP ist der substituierte Glycerinrest des Lipids durch einen myo-Inositolrest substituiert.

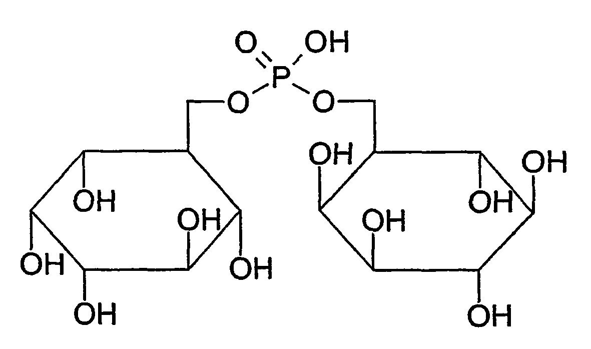

Di-myo-Inositolphosphat trägt die Strukturmotive klassischer Stabilisatoren (viele OH-Gruppen, Phosphat) und vereint somit in idealer Weise zwei wichtige Stabilisierungsmechanismen:
- Bindung hydrophiler Proteindomainen über **OH-Gruppen (Simulation von Wasser)**, Verhinderung von Wasserstoffbrückenbildung innerhalb der Proteinstruktur und zwischen Proteinen während der Trocknung und Rehydrierung.
- Präsentation eine **Phosphatanions:** Stabilisierung von Proteinseitenketten über ionische und van-der-Waals Wechselwirkungen Desweiteren zeichnen folgende Merkmale DIP aus:
   - Hohe chemische und physikalische Stabilität
   - pH-Stabilität
   - Lichtstabilität
   - Farblos
   - Sehr gute Löslichkeit in Wasser
   - kein zu erwartendes allergenes Potential (Substanz aus Extremophilen)

Letztendlich darf die solubilisierende Wirkung von DIP als Ersatz des Wasser in der Zelle nicht vergessen werden. DIP ist wie die anderen Kompatiblen Solute das natürliche Lösungsmittel der Zelle. Um ihre Wirkung in der Zelle überhaupt entfalten zu können, müssen biogene Stoffe solubilisiert werden.

| | **Diinositolphosphat** |
|---|---|
| **Molekulargewicht** | 422,27 g/mol |
| **Summenformel** | C₁₂H₂₃O₁₄P |
| **Erscheinungsform** | farbloser Feststoff |
| **CAS-Nummer** | 143491-08-1 |
| **Konzentrationsoptimum** | 250 - 400 mM |

Entscheidend für die stabilisierende Wirkung des optisch aktiven DIP ist, daß die Substanz als Kaliumsalz vorliegt.

### Aktiver Bestandteil von Liposomenstrukturen

Phospholipide sind in der Lage, sich in Form von zweidimensionalen Lamellen zu Lipidbläschen, den sogenannten Liposomen zusammenzuschließen, in deren Zentrum wasserlösliche Komponenten eingebracht werden können. Da sich Liposomen aufgrund Ihrer gleichartigen Natur mit Zellmembranen verschmelzen lassen, ist es möglich, Wirkstoffe mit Hilfe von Liposomen in die Zelle einzuschleusen. Die Stabilisierung dieser Lipidstrukturen bei Lagerung und kosmetischem Einsatz ist daher von großer Bedeutung. Aufgrund seiner verwandten Struktur zu den Lipiden ist hier der Einsatz des kompatiblen Solutes Di-myo-Inositolphosphat als stabilisierendes Agens interessant.

### Thermoprotektor von Proteinen

Die hervorragenden Eigenschaften von Di-myo-Inositolphosphat bei der Stabilisierung von Proteinen und Enzymen unter verschiedenen Streßbedingungen und die strukturelle Verwandtschaft zu den elementaren Bausteinen von Zellmembran und Membranen verschiedener Zellorganellen machen dieses Kompatible Solut besonders für den Einsatz im Bereich der Kosmetik interessant.

Proteine und Peptide als Wirkstoffe gewinnen auch in der Kosmetik/Dermatologie eine immer größere Bedeutung. Beispiele für derartige Applikationen in der Kosmetik sind z. B. Kollagenpräparationen, der Einsatz von Cytokinen bei antientzündlich wirkenden Präparaten, der Schutz des sog. Mikroproteins oder auch von Reparaturenzymen der Haut.

Die hautverträgliche Stabilisierung und Erhaltung der Funktionalität dieser Präparate gegenüber Temperaturdenaturierung und Proteolyse ist entscheidend für den Anwendungserfolg. Die funktionale Stabilisierung von Proteinen und Peptiden in den Präparationen (Lagerung, Transport) aber auch nach der Applikation auf der Haut (Depotwirkung) ist von großer Bedeutung. Insbesondere auf der Haut sind viele "proteinfeindliche" Abwehrmechanismen installiert (z. B. Proteasen), die den Erfolg einer proteingestützten kosmetischen Applikation gefährden.

Über die generelle Wirkung von DIP ist zur Zeit wenig bekannt. Dies liegt im Wesentlichen an der bislang zu geringen Verfügbarkeit der Verbindung. In ersten Experimenten konnte gezeigt werden, daß DIP sehr gute und im Vergleich mit anderen kompatiblen Soluten um Dimensionen bessere Schutzwirkung bei der thermisch bedingten Denaturierung von Proteinen besitzt.

Denaturierungs- und damit kausal verbundene Alterungsprozesse verlaufen immer nach einer Funktion, die von den Faktoren Zeit und Temperatur abhängig ist. Temperaturbedingte Denaturierung läuft somit nicht nur bei außergewöhnlich hohen Temperaturen, sondern auch bei physiologischen Temperaturen ab. Jede Steigerung der Temperaturstabilität bedeutet somit eine Verlangsamung von Denaturierungs- und Alterungsprozessen. Wirkstoffe die auf diese Weise gegen das sogenannte "skin-aging" schützen sind für die Kosmetikindustrie von enormem Interesse.

### DGP:

Der Vergleich des kompatiblen Solutes Di-Glycerinphosphat (DGP) mit den zuvor beschriebenen Glycerophospholipiden zeigt deutlich eine starke strukturelle Nähe der Verbindungen. Mit R1, R2 = H und R3 = 2,3-Propandiol liegt im DGP die einfachste Struktur eines Phosphodiesters mit Glycerin als Alkoholkomponente vor (s. folgende Abbildung).

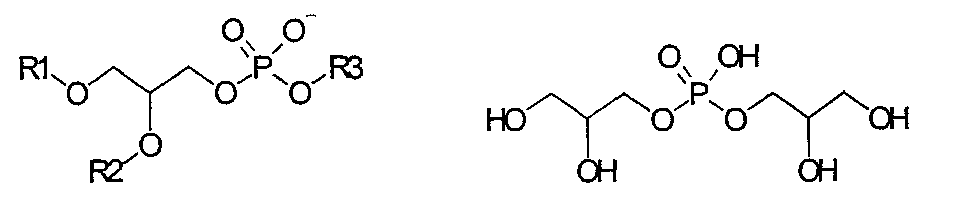

Nach heutigem Erkenntnisstand dürften im wesentlichen zwei Effekte für das Stabilisierungspotential des DGP verantwortlich sein:
- Bindung hydrophiler Proteindomäinen über **OH-Gruppen (Simulation von Wasser)**, Verhinderung von Wasserstoffbrückenbildung innerhalb der Proteinstruktur und zwischen Proteinen während der Trocknung und Rehydrierung.
- Präsentation eines **Phosphatanions:** Stabilisierung von Proteinseitenketten über ionische und van-der-Waals Wechselwirkungen.

Desweiteren zeichnen folgende Merkmale DGP aus:
- Hohe chemische und physikalische Stabilität
- pH-Stabilität
- Lichtstabilität
- Farblos
- Sehr gute Löslichkeit in Wasser
- Hohe Biokompatibilität
- Natürlicher Schutzstoff aus R1 Mikroorganismen
- kein zu erwartendes allergenes Potential (Substanz aus Extremophilen)

Letztendlich darf die solubilisierende Wirkung von DGP als Ersatz des Wasser in der Zelle nicht vergessen werden. DGP ist wie die anderen Kompatiblen Solute das natürliche Lösungsmittel der Zelle. Um ihre Wirkung in der Zelle überhaupt entfalten zu können, müssen biogene Stoffe solubilisiert werden.

| | **1,1-Di-Glycerinphosphat** |
|---|---|
| **Molekulargewicht** | 246,15 |
| **Summenformel** | C₆H₁₅O₈P |
| **Erscheinungsform** | farblose Flüssigkeit, freie Säure |
| **CAS-Nummer** | 6418-92-4 |
| **Konzentrationsoptimum** | 100 - 200 mM |

### Feuchtigkeitsschutz

Die Verbindung DGP ist durch ihre starke Wasserbindungsfähigkeit, die durch die ausgeprägte Neigung der zahlreichen Hydroxygruppen im Molekül zur Ausbildung von Wasserstoffbrücken gekennzeichnet, so daß sie als Regulator des Feuchtigkeitshaushaltes der Haut von Interesse ist.

### Liposomen- und Membranschutz

Liposomen gewinnen als hautgängige Vehikel z. B. für Wirkstoffe nicht nur in der Kosmetik immer mehr an Bedeutung. Liposomen sind artifizielle Lipiddoppellayer - künstliche Membranen -, deren Stabilität jedoch begrenzt sind. Zur besseren Lagerung und Transport Bedarf es der Stabilisierung von Liposomenpräparationen, z. B. gegenüber Temperaturstreß und osmotischem Streß. Nur so kann letztendlich ein optimaler Wirkstoffeinsatz über Liposomen gewährleistet werden.

Membranen stellen die natürlichen Barrieren von Zellen und Zellorganellen zum diese Zellen umgebenden Medium dar. Erst Membranen gewährleisten, daß in den einzelnen Zellen bzw. Zellbestandteilen ein konstantes inneres Milieu besteht. Biologische Membranen sind Mehrkomponentensysteme. Die genaue Zusammensetzung einzelner Membranen unterliegt je nach ihrer Herkunft mehr oder weniger großen Schwankungsbreiten. Elementare Bestandteile von Membranen sind jedoch Lipide und Proteine.

Phospholipide sind in der Lage, sich in Form von zweidimensionalen Lamellen zu Lipidbläschen, den sogenannten Liposomen zusammenschließen, in deren Zentrum wasserlösliche Komponenten eingebracht werden können. Die Stabilisierung dieser Lipidstrukturen bei Lagerung und kosmetischem Einsatz ist daher von großer Bedeutung. Aufgrund seiner verwandten Struktur zu den Lipiden ist hier der Einsatz des kompatiblen Solutes Di-Glycerinphosphat als stabilisierendes Agens interessant. Dies wird noch durch die Tatsache untermauert, daß DGP als Bestandteil von Lipid-Hydrolysaten NMR-spektroskopisch nachgewiesen werden konnte.

### Proteinschutz

Proteine und Peptide als Wirkstoffe gewinnen auch in der Kosmetik eine immer größere Bedeutung. Beispiele für derartige Applikationen in der Kosmetik sind z. B. Kollagenpräparationen, der Einsatz von Cytokinen bei antientzündlich wirkenden Präparaten, der Schutz des sog. Mikroproteins oder von Regenerationsenzymen der Haut.

Die hautverträgliche Stabilisierung und Erhaltung der Funktionalität dieser Präparate gegenüber Temperaturdenaturierung und Proteolyse ist entscheidend für den Anwendungserfolg. Die funktionale Stabilisierung von Proteinen und Peptiden in den Präparationen (Lagerung, Transport) aber auch nach der Applikation auf der Haut (Depotwirkung) ist von großer Bedeutung. Insbesondere auf der Haut sind viele "proteinfeindliche" Abwehrmechanismen installiert (z. B. Proteasen), die den Erfolg einer proteingestützten kosmetischen Applikation gefährden.

### Gewerbliche Anwendung der Erfindung

Die gewerbliche Anwendung der Erfindung liegt im gesamten Bereich der kosmetischen und dermatologischen Produkte sowie als Forschungsreagenzien im Bereich der kosmetischen und dermatologischen Entwicklung. Ebenso liegt eine gewerbliche Anwendung in der postoperativen Behandlung der Haut (z. B.. Wundversorgung zur Unterstützung des Heilungsprozesses) und im Zusatz der Verbindungen zur Pflastern, Masken und Verbänden die auf die Haut aufgebracht werden.

Mögliche weitere Anwendungen der Verbindungen in der Kosmetik und Dermatologie sind z. B.:
- Mikroeinkapselung kosmetischer Wirkstoffe und Additive
- Liposomenstabilisierung
- Feuchtigkeitsspender
- Radikal- und UV-Schutz
- Schutz vor Umweltgiften und Salzen
- Osmostressprotektantien

### Weg zur Ausführung der Erfindung inkl. Beispiele

Die Herstellung der kosmetischen und dermatologischen Formulierungen erfolgt, indem eine oder mehrere der erfindungsgemäßen Verbindungen der Formeln Ia (Diinositolphosphat) oder Ib (Di-Glycerinphosphat), deren physiologisch verträgliche Salze, stereoisomere Formen oder Derivate, gegebenenfalls mit Hilfs- und/oder Trägerstoffen in eine geeignete Formulierungsform gebracht werden.

Die Hilfs- und Trägerstoffe stammen aus der Gruppe der Trägermittel, Konservierungsstoffe und anderer üblicher Hilfsstoffe. Die in kosmetischen und dermatologischen Formulierungen enthaltenen Verbindungen der Formel Ia (Diinositolphosphat), den physiologisch verträglichen Salzen der erfindungsgemäßen Verbindungen und deren stereoisomere Formen werden äußerlich angewendet. Als Anwendungsform seien z. B. genannt: Lösungen, Suspensionen, Emulsionen, Pasten, Salben, Gele, Cremes, Lotionen, Puder, Seifen, tensidhaltige Reinigungspräparate, Öle und Sprays. Zusätzlich können den Formulierungen beliebige übliche Trägerstoffe, Hilfsstoffe und gegebenenfalls weitere Wirkstoffe zugesetzt werden. Vorzuziehende Hilfsstoffe stammen aus der Gruppe der Konservierungsstoffe, Antioxidantien, Stabilisatoren, Lösungsvermittler, Vitamine, Färbemittel und Geruchsverbesserer.

Salben, Pasten, Cremes und Gele können neben einer oder mehrere Verbindungen ausgewählt aus den erfindungsgemäßen Verbindungen, die übliche Trägerstoffe enthalten, z. B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können neben einer oder mehrere der erfindungsgemäßen Verbindungen zusätzlich zu den üblichen Trägerstoffen die üblichen Treibmittel, z. B. Chlorfluorkohlenwasserstoffe, Propan/Butan oder Dimethylether, enthalten.

Lösungen und Emulsionen können neben einer oder mehreren der erfindungsgemäßen Verbindungen zusätzlich die üblichen Trägerstoffen wie Lösungsmittel, Lösungsvermittler und Emulgatoren oder Öle enthalten.

Suspensionen können neben einer oder mehrerer der erfindungsgemäßen Verbindungen zusätzliche Trägerstoffe wie z. B. Wasser oder Ethanol enthalten.

Weitere Applikationsformen sind z. B. Seifen, tensidhaltige Reinigungsmittel, Gesichts- und Körperöle, Lippenstifte, Lippenpflegestifte, Mascara, Eyeliner, Lidschatten, Rouge, Puder-, Emulsions- und Wachs-Make up sowie Sonnenschutz-, Prä-Sun- und After-Sun-Präparate.

Der Anteil der erfindungsgemäßen Verbindungen, deren physiologisch verträglicher Salze, stereoisomeren Formen an den kosmetischen und dermatologischen Formulierungen beträgt vorzugsweise 0,0001 bis 50 Gew.-%, besonders bevorzugt von 0,001 bis 10 Gew.-% bezogen auf die gesamte kosmetische Formulierung.

Die Applikation von DIP und DGP unterstützt z. B. durch die nachgewiesene Stabilisierung gegen thermische Proteindenaturierung und -inaktivierung das zelleigene antioxidative Potential. Somit wird einer Schädigung durch Radikale entgegengewirkt. Durch den nachgewiesenen DNA-Schutz kompatibler Solute und deren proteinstabilisierende Wirkung stellt der erfindungsgemäße Einsatz von DIP oder DGP eine neue Art des aktiven Sonnenschutzes (z. B. UV- und IR-Strahlung) in der Kosmetik und Dermatologie dar.

Die nachfolgenden Beispiele sollen die vorliegende Erfindung verdeutlichen, ohne sie einzuschränken.

### Beispiel 1: O/W-Lotion

Aus folgenden Komponenten wird eine erfindungsgemäße Lotion (O/W) enthaltend Diinositolphosphat hergestellt:

| **Komponente** | **Anteil in Gewichtsprozent (% w/v)** |
|---|---|
| Paraffinöl (DAB 9) | 8,00 |
| Isopropylpalmitat | 3,00 |
| Petrofatum | 4,00 |
| Cetylstearylalkohol | 2,00 |
| PEG 40 Rizinusöl | 0,50 |
| Natriumcetylsearylsulfat | 0,50 |
| Natrium Carbomer | 0,40 |
| Diinositolphosphat | 0,50 |
| Glycerin | 3,00 |
| α-Tocopherol | 0,20 |
| Octylmethooxycinnamat | 5,00 |
| Butylmethoxydibenzoylmethan | 1,00 |
| Konservierungsmittel, Farbstoffe, Parfüm | q.s. |
| Wasser | Ad 100,00 |

Als Konservierungsmittel können 0,05 % Propyl-hydroxybenzoat oder 0,15 % Methyl-4-hydroxybenzoat verwendet werden

### Beispiel 2: O/W-Creme

Aus folgenden Komponenten wird eine erfindungsgemäße Creme (O/W) enthaltend Diinositolphosphat hergestellt:

| **Komponente** | **Anteil in Gewichtsprozent (% w/v)** |
|---|---|
| Paraffinöl (DAB 9) | 7,00 |
| Avocadoöl | 3,00 |
| Glycerylmonostearat | 4,00 |
| Diinositolphosphat | 0,50 |
| Titandioxid | 1,00 |
| Natriumlactat | 3,00 |
| Glycerin | 3,00 |
| Konservierungsmittel, Farbstoffe, Parfüm | q.s. |
| Wasser | Ad 100,00 |

Als Konservierungsmittel können 0,05 % Propyl-hydroxybenzoat oder 0,15 % Methyl-4-hydroxybenzoat verwendet werden

### Beispiel 3: Liposomenhaltiges Gel

Aus folgenden Komponenten wird ein erfindungsgemäßes liposomenhaltiges Gel enthaltend Diinositolphosphat hergestellt:

| **Komponente** | **Anteil in Gewichtsprozent (% w/v)** |
|---|---|
| Lecithin | 6,00 |
| Schibutter | 3,00 |
| Diinositolphosphat | 0,50 |
| α-Tocopherol | 0,20 |
| Biotin | 0,08 |
| Natriumcitrat | 0,50 |
| Glycin | 0,20 |
| Harnstoff | 0,20 |
| Natrium PCA | 0,50 |
| Hydrolysiertes Kollagen | 2,00 |
| Xanthan Gummi | 1,40 |
| Sorbitol | 3,00 |
| Konservierungsmittel, Farbstoffe, Parfüm | q.s. |
| Wasser | ad 100,00 |

Als Konservierungsmittel können 0,05 % Propyl-hydroxybenzoat oder 0,15 % Methyl-4-hydroxybenzoat verwendet werden

### Beispiel 4: Gel

Aus folgenden Komponenten wird ein erfindungsgemäßes Gel (O/W) enthaltend Diinositolphosphat hergestellt:

| **Komponente** | **Anteil in Gewichtsprozent (% w/v)** |
|---|---|
| Carbopol | 2,00 |
| Triethanolamin | 3,00 |
| Diinositolphosphat | 0,50 |
| α-Tocopherylacetat | 0,20 |
| Polyoxyethylensorbitanfettsäureester (Tween 20) | 0,50 |
| Glycerin | 2,00 |
| Natrium PCA | 0,50 |
| Hydrolysiertes Kollagen | 2,00 |
| Konservierungsmittel, Farbstoffe, Parfüm | q.s. |
| Wasser | ad 100,00 |

Als Konservierungsmittel können 0,05 % Propyl-hydroxybenzoat oder 0,15 % Methyl-4-hydroxybenzoat verwendet werden

### Beispiel 5: Sonnenschutzemulsion

Aus folgenden Komponenten wird eine erfindungsgemäße Sonnenschutzemulsion enthaltend Diinositolphosphat hergestellt:

| **Komponente** | **Anteil in Gewichtsprozent (% w/v)** |
|---|---|
| Cyclomethicon | 2,00 |
| Cetyldimethicon Copolyol | 0,20 |
| PEG 22-Dodecyl Copolymer | 3,00 |
| Paraffinöl (DAB 9) | 2,00 |
| Caprylsäure-/Caprinsäure Triglycerid | 5,80 |
| Octylmethoxycinnamat | 5,80 |
| Butyl-methoxy-dibenzoylmethan | 4,00 |
| α-Tocopherylacetat | 0,50 |
| ZnSO4 | 0,70 |
| Na3IIEDTA | 0,30 |
| Diinositolphosphat | 0,50 |
| Konservierungsmittel, Farbstoffe, Parfüm | q.s. |
| Wasser | ad 100,00 |

Als Konservierungsmittel können 0,05 % Propyl-hydroxybenzoat oder 0,15 % Methyl-4-hydroxybenzoat verwendet werden.

### Beispiel 6: Sonnenlotion

Aus folgenden Komponenten wird eine erfindungsgemäße emulgatorfreie Sonnenlotion SPF 30 (W/O) enthaltend Diinositolphosphat hergestellt:

| **Komponente** | **Anteil in Gewichtsprozent (% w/v)** |
|---|---|
| Caprylsäure-/Caprinsäure Triglyceride | 30,00 |
| Uvinul T150 | 4,0 |
| Eusolex 6300 | 2,00 |
| Neo Heliopan OS | 5,00 |
| Parsol 1789 | 2,00 |
| Eusolex T2000 | 4,00 |
| Aerosil R972 | 2,00 |
| Zinkoxid | 2,5 |
| Natrosol Plus 330CS | 0,5 |
| Glycerin | 10,0 |
| Diinositolphosphat | 0,50 |
| Konservierungsmittel, Farbstoffe, Parfüm | q.s. |
| Wasser | ad 100,00 |

Als Konservierungsmittel können 0,05 % Propyl-hydroxybenzoat oder 0,15 % Methyl-4-hydroxybenzoat verwendet werden.

### Beispiel 7: Haarwasser

Aus folgenden Komponenten wird ein erfindungsgemäßes Haarwasser enthaltend Diinositolphosphat hergestellt:

| **Komponente** | **Anteil in Gewichtsprozent (% w/v)** |
|---|---|
| Ethanol | 40,00 |
| Diisopropyladipat | 0,10 |
| α-Tocopherylacetat | 0,50 |
| Diinositolphosphat | 0,50 |
| Konservierungsmittel, Farbstoffe, Parfüm | q.s. |
| Wasser | ad 100,00 |

Als Konservierungsmittel können 0,05 % Propyl-hydroxybenzoat oder 0,15 % Methyl-4-hydroxybenzoat verwendet werden.

### Beispiel 8: Sprayformulierung

Aus folgenden Komponenten wird eine erfindungsgemäße Sprayformulierung enthaltend Diinositolphosphat hergestellt:

| **Komponente** | **Anteil in Gewichtsprozent (% w/v)** |
|---|---|
| Ethanol | 28,2 |
| α-Tocopherylacetat | 0,10 |
| Diinositolphosphat | 0,50 |
| Konservierungsmittel, Farbstoffe, Parfüm | q.s. |
| Propan/Butan 25/75 | Ad 100,00 |

Als Konservierungsmittel können 0,05 % Propyl-hydroxybenzoat oder 0,15 % Methyl-4-hydroxybenzoat verwendet werden.

### Beispiel 9: O/W-Lotion

Aus folgenden Komponenten wird eine erfindungsgemäße Lotion (O/W) enthaltend Di-Glycerinphosphat hergestellt:

| **Komponente** | **Anteil in Gewichtsprozent (% w/v)** |
|---|---|
| Paraffinöl (DAB 9) | 8,00 |
| Isopropylpalmitat | 3,00 |
| Petrofatum | 4,00 |
| Cetylstearylalkohol | 2,00 |
| PEG 40 Rizinusöl | 0,50 |
| Natriumcetylsearylsulfat | 0,50 |
| Natrium Carbomer | 0,40 |
| Di-Glycerinphosphat | 0,50 |
| Glycerin | 3,00 |
| α-Tocopherol | 0,20 |
| Octylmethooxycinnamat | 5,00 |
| Butylmethoxydibenzoylmethan | 1,00 |
| Konservierungsmittel, Farbstoffe, Parfüm | q.s. |
| Wasser | Ad 100,00 |

Als Konservierungsmittel können 0,05 % Propyl-hydroxybenzoat oder 0,15 % Methyl-4-hydroxybenzoat verwendet werden.

### Beispiel 10: O/W-Creme

Aus folgenden Komponenten wird eine erfindungsgemäße Creme (O/W) enthaltend Di-Glycerinphosphat hergestellt:

| **Komponente** | **Anteil in Gewichtsprozent (% w/v)** |
|---|---|
| Paraffinöl (DAB 9) | 7,00 |
| Avocadoöl | 3,00 |
| Glycerylmonostearat | 4,00 |
| Di-Glycerinphosphat | 0,50 |
| Titandioxid | 1,00 |
| Natriumlactat | 3,00 |
| Glycerin | 3,00 |
| Konservierungsmittel, Farbstoffe, Parfüm | q.s. |
| Wasser | Ad 100,00 |

Als Konservierungsmittel können 0,05 % Propyl-hydroxybenzoat oder 0,15 % Methyl-4-hydroxybenzoat verwendet werden.

### Beispiel 11: Liposomenhaltiges Gel

Aus folgenden Komponenten wird ein erfindungsgemäßes liposomenhaltiges Gel enthaltend Di-Glycerinphosphat hergestellt:

| **Komponente** | **Anteil in Gewichtsprozent (% w/v)** |
|---|---|
| Lecithin | 6,00 |
| Schibutter | 3,00 |
| Di-Glycerinphosphat | 0,50 |
| a-Tocopherol | 0,20 |
| Biotin | 0,08 |
| Natriumcitrat | 0,50 |
| Glycin | 0,20 |
| Harnstoff | 0,20 |
| Natrium PCA | 0,50 |
| Hydrolysiertes Kollagen | 2,00 |
| Xanthan Gummi | 1,40 |
| Sorbitol | 3,00 |
| Konservierungsmittel, Farbstoffe, Parfüm | q.s. |
| Wasser | ad 100,00 |

Als Konservierungsmittel können 0,05 % Propyl-hydroxybenzoat oder 0,15 % Methyl-4-hydroxybenzoat verwendet werden.

### Beispiel 12: Gel

Aus folgenden Komponenten wird ein erfindungsgemäßes Gel (O/W) enthaltend Di-Glycerinphosphat hergestellt:

| **Komponente** | **Anteil in Gewichtsprozent (% w/v)** |
|---|---|
| Carbopol | 2,00 |
| Triethanolamin | 3,00 |
| Di-Glycerinphosphat | 0,50 |
| a-Tocopherylacetat | 0,20 |
| Polyoxyethylensorbitanfettsäureester (Tween 20) | 0,50 |
| Glycerin | 2,00 |
| Natrium PCA | 0,50 |
| Hydrolysiertes Kollagen | 2,00 |
| Konservierungsmittel, Farbstoffe, Parfüm | q.s. |
| Wasser | ad 100,00 |

Als Konservierungsmittel können 0,05 % Propyl-hydroxybenzoat oder 0,15 % Methyl-4-hydroxybenzoat verwendet werden.

### Beispiel 13: Sonnenschutzemulsion

Aus folgenden Komponenten wird eine erfindungsgemäße Sonnenschutzemulsion enthaltend Di-Glycerinphosphat hergestellt:

| **Komponente** | **Anteil in Gewichtsprozent (% w/v)** |
|---|---|
| Cyclomethicon | 2,00 |
| Cetyldimethicon Copolyol | 0,20 |
| PEG 22-Dodecyl Copolymer | 3,00 |
| Paraffinöl (DAB 9) | 2,00 |
| Caprylsäure-/Caprinsäure Triglycerid | 5,80 |
| Octylmethoxycinnamat | 5,80 |
| Butyl-methoxy-dibenzoylmethan | 4,00 |
| α-Tocopherylacetat | 0,50 |
| ZnSO4 | 0,70 |
| Na3IIEDTA | 0,30 |
| Di-Glycerinphosphat | 0,50 |
| Konservierungsmittel, Farbstoffe, Parfüm | q.s. |
| Wasser | ad 100,00 |

Als Konservierungsmittel können 0,05 % Propyl-hydroxybenzoat oder 0,15 % Methyl-4-hydroxybenzoat verwendet werden.

### Beispiel 14: Sonnenlotion

Aus folgenden Komponenten wird eine erfindungsgemäße emulgatorfreie Sonnenlotion SPF 30 (W/O) enthaltend Di-Glycerinphosphat hergestellt:

| **Komponente** | **Anteil in Gewichtsprozent (% w/v)** |
|---|---|
| Caprylsäure-/Caprinsäure Triglyceride | 30,00 |
| Uvinul T150 | 4,0 |
| Eusolex 6300 | 2,00 |
| Neo Heliopan OS | 5,00 |
| Parsol 1789 | 2,00 |
| Eusolex T2000 | 4,00 |
| Aerosil R972 | 2,00 |
| Zinkoxid | 2,5 |
| Natrosol Plus 330CS | 0,5 |
| Glycerin | 10,0 |
| Di-Glycerinphosphat | 0,50 |
| Konservierungsmittel, Farbstoffe, Parfüm | q.s. |
| Wasser | ad 100,00 |

Als Konservierungsmittel können 0,05 % Propyl-hydroxybenzoat oder 0,15 % Methyl-4-hydroxybenzoat verwendet werden.

### Beispiel 15: Haarwasser

Aus folgenden Komponenten wird ein erfindungsgemäßes Haarwasser enthaltend Di-Glycerinphosphat hergestellt:

| **Komponente** | **Anteil in Gewichtsprozent (% w/v)** |
|---|---|
| Ethanol | 40,00 |
| Diisopropyladipat | 0,10 |
| α-Tocopherylacetat | 0,50 |
| Di-Glycerinphosphat | 0,50 |
| Konservierungsmittel, Farbstoffe, Parfüm | q.s. |
| Wasser | ad 100,00 |

Als Konservierungsmittel können 0,05 % Propyl-hydroxybenzoat oder 0,15 % Methyl-4-hydroxybenzoat verwendet werden.

### Beispiel 16: Sprayformulierung

Aus folgenden Komponenten wird eine erfindungsgemäße Sprayformulierung enthaltend Di-Glycerinphosphat hergestellt:

| **Komponente** | **Anteil in Gewichtsprozent (% w/v)** |
|---|---|
| Ethanol | 28,2 |
| α-Tocopherylacetat | 0,10 |
| Di-Glycerinphosphat | 0,50 |
| Konservierungsmittel, Farbstoffe, Parfüm | q.s. |
| Propan/Butan 25/75 | Ad 100,00 |

Als Konservierungsmittel können 0,05 % Propyl-hydroxybenzoat oder 0,15 % Methyl-4-hydroxybenzoat verwendet werden.

## Patentansprüche

1. Verwendung von Di-myo-Inositolphosphat und/oder Di-Glycerinphosphat, ihrer physiologisch verträglichen Salze, stereoisomeren Formen und/oder Derivate zur Herstellung einer kosmetischen oder dermatologischen Formulierung zum Schutz menschlicher Hautzellen vor UV-Strahlung und/oder IR-Strahlung.

2. Verwendung nach Anspruch 1, **dadurch gekennzeichnet, dass** die Verbindungen, ihre physiologisch verträglichen Salze, stereoisomeren Formen und/oder Derivate in Konzentrationen von 0,01 bis 50 Gew.-%, bevorzugt 0,05 bis 10 Gew.-%, insbesondere 0,1 bis 5 Gew.-% bezogen auf das Gesamtgewicht der Formulierung vorliegen.

3. Verwendung nach Anspruch 1 oder 2, **dadurch gekennzeichnet, dass** die kosmetische oder dermatologische Formulierung einen oder mehrere UV-Filter enthält.

4. Verwendung nach einem der Ansprüche 1 bis 3, **dadurch gekennzeichnet, dass** die kosmetische oder dermatologische Formulierung eine oder mehrere Substanzen ausgewählt aus Enzymen, Vitaminen und Vitamin-Derivaten enthält.

5. Verwendung nach einem der Ansprüche 1 bis 4, **dadurch gekennzeichnet, dass** die Formulierung in Form einer Lösung, einer Supension, einer Emulsion, einer Paste, einer Salbe, eines Gels, einer Creme, einer Lotion, eines Puders, einer Seife, eines tensidhaltigen Reinigungspräparats, eines Öls, eines Lippenstifts, eines Lippenpflegestifts, einer Mascara, eines Eyeliners, von Lidschatten, von Rouge, eines Puder-, Emulsions- oder Wachs-Make ups, eines Sonnenschutz-, Prä-Sun- oder After-Sun-Präparats, eines Haarwassers, eines Pflasters, eines Verbands oder eines Sprays vorliegt.

6. Formulierung, enthaltend Di-myo-Insositolphosphat und/oder Di-Glycerinphosphat, ihre physiologisch verträglichen Salze, stereoisomeren Formen und/oder Derivate, zum Schutz menschlicher Hautzellen vor UV-Strahlung und/oder IR-Strahlung.

7. Formulierung nach Anspruch 6, **dadurch gekennzeichnet, dass** das Di-myo-Inositolphosphat und/oder Di-Glycerinphosphat, ihre physiologisch verträglichen Salze, stereoisomeren Formen und/oder Derivate in einer Konzentration von 0,01 bis 50 Gew.-%, bevorzugt 0,05 bis 10 Gew.-%, insbesondere 0,1 bis 5 Gew.-% bezogen auf das Gesamtgewicht der Formulierung vorliegt.

8. Formulierung nach Anspruch 6 oder 7, **dadurch gekennzeichnet, dass** die Formulierung einen oder mehrere UV-Filter enthält.

9. Formulierung nach einem der Ansprüche 6 bis 8, **dadurch gekennzeichnet, dass** die Formulierung eine oder mehrere Substanzen ausgewählt aus Enzymen, Vitaminen und Vitamin-Derivaten enthält.

10. Formulierung nach einem der Ansprüche 6 bis 9, **dadurch gekennzeichnet, dass** die Formulierung in Form einer Lösung, einer Supension, einer Emulsion, einer Paste, einer Salbe, eines Gels, einer Creme, einer Lotion, eines Puders, einer Seife, eines tensidhaltigen Reinigungspräparats; eines Öls, eines Lippenstifts, eines Lippenpflegestifts, einer Mascara, eines Eyeliners, von Lidschatten, von Rouge, eines Puder-, Emulsions- oder Wachs-Make ups, eines Sonnenschutz-, Prä-Sun- oder After-Sun-Präparats, eines Haarwassers, eines Pflasters, eines Verbands oder eines Sprays vorliegt.

## Claims

1. The use of Di-myo-inositol phosphate and/or Diglycerine phosphate, their physiologically compatible salts, stereoisomeric forms and/or derivatives for producing a cosmetic or dermatological formulation for protecting human skin cells from UV radiation and/or IR radiation.

2. The use as claimed in Claim 1, **characterised in that** the compounds, their physiologically compatible salts, stereoisomeric forms and/or derivatives are present in concentrations of 0.01 to 50wt.%, preferably 0.05 to 10wt.%, particularly 0.1 to 5wt.%, with respect to the total weight of the formulation.

3. The use as claimed in Claim 1 or 2, **characterised in that** the cosmetic or dermatological formulation contains one or more UV filters.

4. The use as claimed in one of Claims 1 to 3, **characterised in that** the cosmetic or dermatological formulation contains one or more substances selected from enzymes, vitamins and vitamin derivatives.

5. The use as claimed in one of Claims 1 to 4, **characterised in that** the formulation is in the form of a suspension, an emulsion, a paste, an ointment, a gel, a cream, a lotion, a powder, a soap, a tenside-containing cleaning preparation, an oil, a lipstick, a lip care stick, a mascara, an eye liner, eye shadow, rouge, a powder, emulsion or wax make-up, a sun protection preparation, a pre-sun or after-sun preparation preparation, hair lotion, a plaster, a dressing, or a spray.

6. A formulation containing Di-myo-inositol phosphate and/or Diglycerine phosphate, their physiologically compatible salts, stereoisomeric forms and/or derivatives for protecting human skin cells from UV radiation and/or IR radiation.

7. A formulation as claimed in Claim 6, **characterised in that** the Di-myo-inositol phosphate and/or Diglycerine phosphate, their physiologically compatible salts, stereoisomeric forms and/or derivatives is present in a concentration of 0.01 to 5-wt.%, preferably 0.05 to 10wt.%, particularly 0.1 to 5wt.%, with respect to the total weight of the formulation.

8. A formulation as claimed in Claim 6 or 7, **characterised in that** the formulation contains one or more UV filters.

9. A formulation as claimed in one of Claims 6 to 8, **characterised in that** the formulation contains one or more substances selected from enzymes, vitamins and vitamin derivatives.

10. A formulation as claimed in on of Claims 6 to 9, **characterised in that** the formulation is in the form of a solution, a suspension, an emulsion, a paste, an ointment, a gel, a cream, a lotion, a powder, a soap, a tenside-containing cleaning preparation, an oil, a lipstick, a lip care stick, a mascara, an eye liner, eye shadow, rouge, a powder, emulsion or wax make-up, a sun protection preparation, a pre-sun preparation or after-sun preparation, hair lotion, a plaster, a dressing, or a spray.

## Revendications

1. Utilisation de di-myo-inositol-phosphate et/ou de di-glycérine-phosphate, de leurs sels physiologiquement compatibles, formes stéréo-isomères et/ou dérivés pour la fabrication d'une formulation cosmétique ou dermatologique destinée à protéger les cellules cutanées humaines contre le rayonnement ultraviolet (UV) et/ou le rayonnement infrarouge (IR).

2. Utilisation selon la revendication 1, **caractérisée en ce que** les composés, leurs sels physiologiquement compatibles, formes stéréo-isomères et/ou dérivés sont présents dans des concentrations de 0,01 à 50 % en poids, de préférence 0,05 à 10 % en poids, en particulier 0,1 à 5 % en poids, rapporté au poids total de la formulation.

3. Utilisation selon la revendication 1 ou 2, **caractérisée en ce que** la formulation cosmétique ou dermatologique contient un ou plusieurs filtres anti-UV.

4. Utilisation selon l'une des revendications 1 à 3, **caractérisée en ce que** la formulation cosmétique ou dermatologique contient une ou plusieurs substances choisies parmi des enzymes, des vitamines et des dérivés vitaminiques.

5. Utilisation selon l'une des revendications 1 à 4, **caractérisée en ce que** la formulation est présente sous la forme d'une solution, d'une suspension, d'une émulsion, d'une pâte, d'un baume, d'un gel, d'une crème, d'une lotion, d'une poudre, d'un savon, d'une préparation de nettoyage contenant des tensioactifs, d'une huile, d'un rouge à lèvres, d'un bâtonnet de soin des lèvres, d'un mascara, d'un eye-liner, d'une ombre pour paupières, d'un fond de teint, d'un maquillage en poudre, en émulsion ou à la cire, d'une préparation solaire, présolaire ou après-solaire, d'une lotion capillaire, d'un pansement, d'un bandage ou d'un spray.

6. Formulation contenant du di-myo-inositol-phosphate et/ou du di-glycérine-phosphate, de leurs sels physiologiquement compatibles, formes stéréo-isomères et/ou dérivés pour la fabrication d'une formulation cosmétique ou dermatologique destinée à protéger les cellules cutanées humaines contre le rayonnement ultraviolet (UV) et/ou le rayonnement infrarouge (IR).

7. Formulation selon la revendication 6, **caractérisée en ce que** le di-myo-inositol-phosphate et/ou le di-glycérine-phosphate, leurs sels physiologiquement compatibles, formes stéréo-isomères et/ou dérivés sont présents en une concentration de 0,01 à 50 % en poids, de préférence 0,05 à 10 % en poids, en particulier 0,1 à 5 % en poids, rapporté au poids total de la formulation.

8. Formulation selon la revendication 6 ou 7, **caractérisée en ce que** la formulation cosmétique ou dermatologique contient un ou plusieurs filtres anti-UV.

9. Formulation selon l'une des revendications 6 à 8, **caractérisée en ce que** la formulation cosmétique ou dermatologique contient une ou plusieurs substances choisies parmi des enzymes, des vitamines et des dérivés vitaminiques.

10. Formulation selon l'une des revendications 6 à 9, **caractérisée en ce que** la formulation est présente sous la forme d'une solution, d'une suspension, d'une émulsion, d'une pâte, d'un baume, d'un gel, d'une crème, d'une lotion, d'une poudre, d'un savon, d'une préparation de nettoyage contenant des tensioactifs, d'une huile, d'un rouge à lèvres, d'un bâtonnet de soin des lèvres, d'un mascara, d'un eye-liner, d'une ombre pour paupières, d'un fond de teint, d'un maquillage en poudre, en émulsion ou à la cire, d'une préparation solaire, présolaire ou après-solaire, d'une lotion capillaire, d'un pansement, d'un bandage ou d'un spray.
